# EUROPEAN PATENT SPECIFICATION

(11) **EP 4 554 631 B1**
(45) Date of publication and mention of the grant of the patent: **12.08.2026**
(21) Application number: 23744708.1
(22) Date of filing: 13.07.2023
(51) Int. Cl.: A61L 9/12

(54) **DEVICE FOR EVAPORATING VOLATILE SUBSTANCES**
VORRICHTUNG ZUM VERDAMPFEN VON FLÜCHTIGEN STOFFEN
DISPOSITIF D'ÉVAPORATION DE SUBSTANCES VOLATILES

(30) Priority: 15.07.2022 ES 202230653
(43) Date of publication of application: 21.05.2025
(73) Proprietor: Zobele Holding SpA, 38100 Trento (IT)
(72) Inventor: SANCHO MARZO, Alberto José, 08019 BARCELONA (ES); GARCÍA NEILA, Carla, 08019 BARCELONA (ES); RAMÍREZ ALVARADO, Jesús Orlando, 08019 BARCELONA (ES)
(74) Representative: Herrero & Asociados, S.L.
(86) International application number: PCT/EP2023/069494
(87) International publication number: WO 2024/013304

(56) References cited:
- EP-A1- 1 516 634
- EP-A1- 1 839 684
- EP-A1- 1 932 546
- EP-A2- 1 331 014
- WO-A1-2004/008889
- US-A1- 2019 117 816
- US-B1- 6 676 033

## Description

### OBJECT OF THE INVENTION

The present invention relates to a device for evaporating volatile substances, which allows the removal of a wick from the device and its replacement with a new wick.

### BACKGROUND OF THE INVENTION

The use of volatile substance refills in volatile substance evaporation devices is well known.

These refills consist of a container containing a liquid in which a porous element or wick is placed. The upper part of the wick extends out of the container, and it is this upper part where the evaporation of the volatile substances takes place, either passively, without any other means to enhance evaporation, or under the action of a heater, fan, nebulizer or any known means of diffusion.

Devices for the evaporation of volatile substances without energy supply, i.e., without batteries or connection to a power supply, are not designed to be reusable, but rather the opposite.

Nowadays, such evaporation devices for volatile substances are designed to prevent users from being able to remove the wick, to prevent the user from refilling the container with any substance that has not been validated for that use, and also for safety reasons, to prevent anyone from drinking the volatile substance once the wick has been removed.

In addition, if the user wants to do so, removing the wick is difficult and messy. The consumer may need tools and the risk of getting his hands dirty with oil containing volatile substances is high.

US2019117816A1 discloses a device for the release, in particular for evaporation, of volatile substances such as fragrances or active substances, has a container for a substance to be released. The wick is assigned a squeezing device with a squeezing element, by which a squeezing region of the wick can be subjected to a squeezing force and/or can be compressed, in particular for a defined short period of time and/or temporarily.

US6676033B1 discloses a dispenser system for scented material for use during hunting includes a container having a sealable opening and a wick stored in the container, the wick having a series of weights distributed thereon. The wick may have a tab positioned on one end to prevent complete removal of the wick from the bottle, and a grab hook at the other end. The dispenser system may include an auxiliary line attachable to the grab hook for connecting the wick to a tree limb of the like.

EP1516634A1 discloses an evaporation apparatus capable of supplying active ingredients sufficiently and stably. One embodiment of the invention comprises a retention vessel keeping a liquid formulation which contains active ingredients, a liquid absorbing mechanism which absorbs the liquid formulation from said retention vessel, an evaporation mechanism which evaporates active ingredients of said liquid formulation absorbed into said liquid absorbing mechanism and adjusting means which adjusts the evaporation of active ingredients.

EP1839684A1 discloses a dispenser with a bottle which receives the liquid fragrance with a closable cap over an opening and has a wick holder projecting outside from the opening in the bottle with a wick fixed on it. The wick dips into the usage of perfume dispenser partly in the liquid fragrance and protrudes partly out of the fragrance. The wick is fixed on the wick holder by means of a clip element. The wick holder has a form extended in a longitudinal direction. The clip element comprises a bracket extended perpendicular to the longitudinal direction of the wick holder.

### DESCRIPTION OF THE INVENTION

Therefore, an objective of the present invention is to provide a device for evaporating volatile substances which allows the wick to be removed and replaced by a new one in a simple manner.

The device for evaporating volatile substances according to the present invention is defined in claim 1. Optional additional features are included in the dependent claims.

In particular, the device for evaporating volatile substances comprises:
- a container in which volatile substances are housed;
- a wick, which absorbs the volatile substances, through which the volatile substances evaporate; and
- a stopper attachable to the container, which can be placed in a closed position, where the evaporation of the volatile substances is prevented, and an evaporation position, where the wick is exposed to the environment and the evaporation of the volatile substances is allowed, and
- a clamping element, movable between a clamping position, in which the clamping element clamps the wick, and a release position, in which the clamping element is separated from the wick.

The clamping element comprises a central body and two elastic arms, each elastic arm comprising a gripping element on its side furthest from the central body, so that pressure on the central body causes the gripping elements to move towards each other.

Preferably, the elastic arms of the clamping element are curved, and the clamping element also comprises a connecting portion between the elastic arms.

According to a preferred embodiment, said connecting portion is elastic and U-shaped.

In addition, the ends of the connecting portion are preferably attached to the ends of the elastic arms furthest away from the central body.

The stopper of the device according to the present invention preferably comprises a central hole, where the clamping element is housed, and the central body of the clamping element is at the same level as the top of the stopper in its release position.

The container preferably comprises a neck, the gripping elements of the clamping element being in contact with the neck in its release position.

The stopper further comprises an inner body, which is located between the wick and the clamping element, the inner body of the stopper being preferably cylindrical in shape.

With the device for evaporating volatile substances according to the present invention it is possible that the different components of the device are properly recycled, e.g., the glass bottle, the wick, the stopper, etc.

In addition, it allows the reuse of part of the components to assemble a new refill, i.e., a new wick and refill the container.

It also ensures the integrity of the wick when it is in use but allows it to be easily and cleanly separated when the wick life is over.

### DESCRIPTION OF THE DRAWINGS

In order to complement the description being made and in order to assist in a better understanding of the features of the invention, in accordance with a preferred example of a practical embodiment thereof, a set of drawings is attached hereto as an integral part of the said description, in which the following is illustratively and non-limitingly depicted:

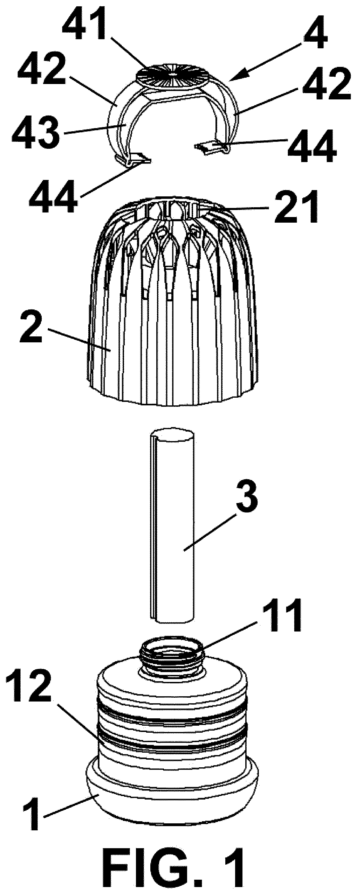

Figure 1 is an exploded perspective view of the device for evaporating volatile substances according to the present invention;
Figure 2 is a perspective section view of the device for evaporating volatile substances according to the present invention with its stopper in its closed position and the clamping element in its release position;
Figure 3 is a perspective section view of the device for evaporating volatile substances according to the present invention with its stopper in its evaporation position and the clamping element in its clamping position; and

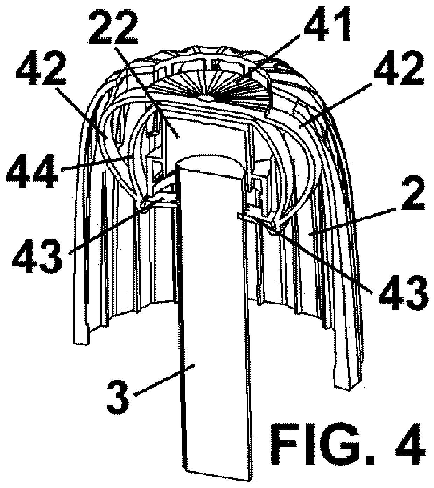

Figure 4 is a perspective section view of the stopper of the device for evaporating volatile substances according to the present invention with the wick attached, removed from the rest of the evaporation device for volatile substances.

### PREFERRED EMBODIMENT OF THE INVENTION

The device for evaporating volatile substances according to a present invention comprises a container (1) in which the volatile substances are housed. This container (1) is, for example, made of glass, and contains within it a liquid containing the volatile substances.

Furthermore, according to the embodiment shown, the container (1) comprises a neck (11) and may comprise threads (12) or ribs for the removable and/or displaceable attachment of a stopper (2).

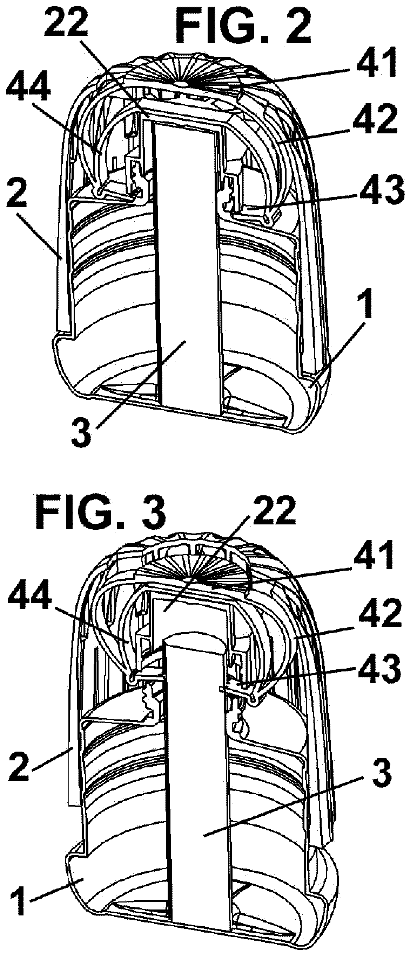

Said stopper (2) is attachable to said container (1), and can be placed in a closing position, shown in figure 2, in which evaporation of the volatile substances is prevented, and an evaporation position, shown in figure 3, in which a wick (3) is exposed to the environment and evaporation of the volatile substances is allowed.

The wick (3) is partially placed inside the container (1) to absorb the volatile substances contained in the container (1), and a part of the wick (3) is placed outside the container (1), through which the volatile substances evaporate into the environment. The wick (3) is made of any porous material suitable to allow the absorption and evaporation of the volatile substances.

The device for evaporating volatile substances also comprises a clamping element (4), which is movable between a clamping position, shown in figure 3, in which the clamping element (4) clamps the wick (3), and a release position, in which the clamping element (4) is detached from the wick (3).

According to the embodiment shown, the clamping element (4) comprises a central body (41) and two elastic arms (42), each elastic arm (42) comprising a gripping element (43) on its side furthest from the central body (41), so that pressure on the central body (41) causes the gripping elements (43) to move towards each other.

The central body (41) is preferably disc-shaped, and the elastic arms (42) of the clamping element (4) are curved.

Furthermore, the clamping element (4) also comprises a connecting portion (44) between the elastic arms (42), which is also elastic, and preferably U-shaped. The ends of said connecting portion (44), according to the embodiment shown, are attached to the ends of the elastic arms (42) furthest away from the central body (41).

Said clamping element (4) is located inside the stopper (2), in a hole (21), preferably circular, located in a position centered on the top of the stopper (2). As can be seen in figure 2, in the closed position of the stopper (2), the central body (41) of the clamping element (4) is flush or at the same level as the top of the stopper (2).

The stopper (2) also comprises an inner body (22), which is located between the wick (3) and the clamping element (4), as can be seen in figures 2 and 3. The inner body (22) of the stopper (2) is preferably cylindrical in shape.

The device for evaporating volatile substances according to the present invention has three possible positions.

The first position is for the transport of the device according to the present invention. In this position, the container (1) is closed by the stopper (2), which is in its closed position, shown in figure 2, fulfilling the function of preventing leakage of the liquid contained inside the container (1) during transport, and the clamping element (4) is in its released position, i.e. it does not contact the wick (3), for example, it may be in contact with the neck (11) of the container (1).

The second position is the evaporation position. For activation, the stopper (2) is moved in relation to the container (1), i.e., it is raised vertically according to the arrangement shown in the figures. In this way, the wick (3) is exposed to the air stream and carries away the volatile substances so that the product is activated.

When the product runs out, the user has the option of removing the wick (3) by activating the clamping element (4). To do this, the central body (41) must be pressed as a button. This movement is transmitted by the elastic arms (42) and/or the connection portion (44), causing the gripping elements (43) to move towards each other, and forces the grip on the wick (3) by these gripping elements (43), which preferably comprise teeth.

Once the clamping element (4) is attached to the wick (3), the stopper (2), together with the clamping element (4) and the wick (3), is completely removed. To do this, the user holds down the central body (41) of the clamping element (4).

The clamping element (4) holds the wick (3) for as long as the user presses the central body (41), allowing it to be transported, for example, to a recycling container. Once the user decides that it is time to release the wick (3), the user stops pressing the central body (41) and the wick (3) is released and falls, for recycling, without the need to touch it with the hands.

The device according to the present invention is then ready to be fitted with a new wick for reuse.

In this way, it is possible to recycle the wick (3) in a controlled manner, by means of a pressing and releasing movement once the pressure is released, without contaminating the hands with the substance contained in the container (1), in a clean and safe manner for the user.

## Claims

1. A device for evaporating volatile substances, comprising:
- a container (1) in which the volatile substances are contained;
- a wick (3), which absorbs volatile substances, through which the volatile substances evaporate; and
- a stopper (2) which is attachable to the container (1) and can be placed in a closed position, in which the evaporation of the volatile substances is prevented, and an evaporation position, in which the wick (3) is exposed to the environment and the evaporation of the volatile substances is allowed,
wherein the device for evaporating volatile substances also comprises a clamping element (4), movable between a clamping position, in which the clamping element (4) clamps the wick (3), so that the stopper (2), together with the clamping element (4) and the wick (3) is completely removed, and a release position, in which the clamping element (4) is separated from the wick (3),
**characterized in that** the clamping element (4) comprises a central body (41) and two elastic arms (42), each elastic arm (42) comprising a gripping element (43) on its side furthest from the central body (41), so that pressure on the central body (41) causes the gripping elements (43) to move towards each other.

2. Device for evaporating volatile substances according to claim 1, wherein the elastic arms (42) of the clamping element (4) are curved.

3. Device for evaporating volatile substances according to claim 1 or 2, wherein the clamping element (4) also comprises a connecting portion (44) between the elastic arms (42).

4. Device for evaporating volatile substances according to claim 3, wherein said connecting portion (44) is U-shaped.

5. Device for evaporating volatile substances according to claim 3 or 4, wherein said connecting portion (44) is elastic.

6. Device for evaporating volatile substances according to any one of claims 3 to 5, wherein the ends of the connecting portion (44) are attached to the ends of the elastic arms (42) further away from the central body (41).

7. Device for evaporating volatile substances according to any one of the preceding claims, wherein the stopper (2) comprises a central hole (21), in which the clamping element (4) is housed.

8. Device for evaporating volatile substances according to claims 1 and 8, wherein the central body (41) of the clamping element (4) is flush as the top of the stopper (2) in its release position.

9. Device for evaporating volatile substances according to claim 1, wherein the container (1) comprises a neck (11), the gripping elements (43) of the clamping element (4) being in contact with said neck (11) in their release position.

10. Device for evaporating volatile substances according to any one of the preceding claims, wherein the stopper (2) comprises an inner body (22), which is located between the wick (3) and the clamping element (4).

11. Device for evaporating volatile substances according to claim 10, wherein the inner body (22) of the stopper (2) is cylindrical in shape.

## Patentansprüche

1. Vorrichtung zum Verdunsten von flüchtigen Stoffen, umfassend:
- einen Behälter (1), in dem die flüchtigen Stoffe enthalten sind;
- einen Docht (3), der flüchtige Stoffe aufsaugt und über den die flüchtigen Stoffe verdunsten; und
- einen Verschluss (2), der an dem Behälter (1) angebracht und in eine geschlossene Stellung, in der die Verdunstung der flüchtigen Stoffe verhindert ist, und eine Verdunstungsstellung, in der der Docht (3) der Umgebung ausgesetzt ist und die Verdunstung der flüchtigen Stoffe möglich ist, gebracht werden kann,
wobei die Vorrichtung zum Verdunsten von flüchtigen Stoffen außerdem ein Klemmelement (4) umfasst, das zwischen einer Klemmstellung, in der das Klemmelement (4) den Docht (3) festklemmt, sodass der Verschluss (2) zusammen mit dem Klemmelement (4) und dem Docht (3) vollständig entfernt wird, und einer Freigabestellung, in der das Klemmelement (4) vom Docht (3) getrennt ist, bewegbar ist,
**dadurch gekennzeichnet, dass** das Klemmelement (4) einen zentralen Körper (41) und zwei elastische Arme (42) umfasst, wobei jeder elastische Arm (42) ein Greifelement (43) auf seiner vom zentralen Körper (41) am weitesten entfernten Seite umfasst, sodass Druck auf den zentralen Körper (41) bewirkt, dass sich die Greifelemente (43) aufeinander zu bewegen.

2. Vorrichtung zum Verdunsten von flüchtigen Stoffen nach Anspruch 1, wobei die elastischen Arme (42) des Klemmelements (4) gekrümmt sind.

3. Vorrichtung zum Verdunsten von flüchtigen Stoffen nach Anspruch 1 oder 2, wobei das Klemmelement (4) außerdem einen Verbindungsabschnitt (44) zwischen den elastischen Armen (42) umfasst.

4. Vorrichtung zum Verdunsten von flüchtigen Stoffen nach Anspruch 3, wobei der Verbindungsabschnitt (44) U-förmig ist.

5. Vorrichtung zum Verdunsten von flüchtigen Stoffen nach Anspruch 3 oder 4, wobei der Verbindungsabschnitt (44) elastisch ist.

6. Vorrichtung zum Verdunsten von flüchtigen Stoffen nach einem der Ansprüche 3 bis 5, wobei die Enden des Verbindungsabschnitts (44) an den weiter vom zentralen Körper (41) entfernten Enden der elastischen Arme (42) angebracht sind.

7. Vorrichtung zum Verdunsten von flüchtigen Stoffen nach einem der vorhergehenden Ansprüche, wobei der Verschluss (2) ein mittiges Loch (21) umfasst, in dem das Klemmelement (4) untergebracht ist.

8. Vorrichtung zum Verdunsten von flüchtigen Stoffen nach den Ansprüchen 1 und 8, wobei der zentrale Körper (41) des Klemmelements (4) in seiner Freigabestellung bündig mit der Oberseite des Verschlusses (2) abschließt.

9. Vorrichtung zum Verdunsten von flüchtigen Stoffen nach Anspruch 1, wobei der Behälter (1) einen Hals (11) umfasst, wobei die Greifelemente (43) des Klemmelements (4) in ihrer Freigabestellung in Kontakt mit dem Hals (11) sind.

10. Vorrichtung zum Verdunsten von flüchtigen Stoffen nach einem der vorhergehenden Ansprüche, wobei der Verschluss (2) einen inneren Körper (22) umfasst, der sich zwischen dem Docht (3) und dem Klemmelement (4) befindet.

11. Vorrichtung zum Verdunsten von flüchtigen Stoffen nach Anspruch 10, wobei der innere Körper (22) des Verschlusses (2) eine zylindrische Form hat.

## Revendications

1. Dispositif pour l'évaporation de substances volatiles, comprenant :
- un récipient (1) dans lequel sont contenues les substances volatiles ;
- une mèche (3) qui absorbe les substances volatiles et à travers laquelle celles-ci s'évaporent ; et
- un bouchon (2) pouvant être fixé au récipient (1) et pouvant être placé dans une position fermée, dans laquelle l'évaporation des substances volatiles est empêchée, et dans une position d'évaporation, dans laquelle la mèche (3) est exposée à l'environnement et l'évaporation des substances volatiles est permise,
dans lequel le dispositif destiné à l'évaporation de substances volatiles comprend également un laquelle l'élément de serrage (4), mobile entre une position de serrage, dans laquelle l'élément de serrage (4) serre la mèche (3), de sorte que le bouchon (2), conjointement avec l'élément de serrage (4) et la mèche (3), soit complètement retiré, et une position de libération, dans laquelle l'élément de serrage (4) est séparé de la mèche (3),
**caractérisé en ce que** l'élément de serrage (4) comprend un corps central (41) et deux bras élastiques (42), chaque bras élastique (42) comportant un élément de préhension (43) sur son côté le plus éloigné du corps central (41), de sorte qu'une pression exercée sur le corps central (41) provoque le rapprochement des éléments de préhension (43).

2. Dispositif pour l'évaporation de substances volatiles selon la revendication 1, dans lequel les bras élastiques (42) de l'élément de serrage (4) sont courbés.

3. Dispositif pour l'évaporation de substances volatiles selon la revendication 1 ou 2, dans lequel l'élément de serrage (4) comprend également une partie de liaison (44) entre les bras élastiques (42).

4. Dispositif pour l'évaporation de substances volatiles selon la revendication 3, dans lequel ladite partie de liaison (44) est en forme de U.

5. Dispositif pour l'évaporation de substances volatiles selon la revendication 3 ou 4, dans lequel ladite partie de liaison (44) est élastique.

6. Dispositif pour l'évaporation de substances volatiles selon l'une quelconque des revendications 3 à 5, dans lequel les extrémités de la partie de liaison (44) sont fixées aux extrémités des bras élastiques (42) les plus éloignées du corps central (41).

7. Dispositif pour l'évaporation de substances volatiles selon l'une quelconque des revendications précédentes, dans lequel le bouchon (2) comprend un trou central (21), dans lequel est logé l'élément de serrage (4).

8. Dispositif pour l'évaporation de substances volatiles selon les revendications 1 et 8, dans lequel le corps central (41) de l'élément de serrage (4) affleure la face supérieure du bouchon (2) dans sa position de libération.

9. Dispositif pour l'évaporation de substances volatiles selon la revendication 1, dans lequel le récipient (1) comprend un col(11), les éléments de préhension (43) de l'élément de serrage (4) étant en contact avec ledit col (11) dans leur position de libération.

10. Dispositif pour l'évaporation de substances volatiles selon l'une quelconque des revendications précédentes, dans lequel le bouchon (2) comprend un corps intérieur (22), qui est situé entre la mèche (3) et l'élément de serrage (4).

11. Dispositif pour l'évaporation de substances volatiles selon la revendication 10, dans lequel le corps intérieur (22) du bouchon (2) est de forme cylindrique.
